# DEMANDE DE BREVET EUROPEEN

(11) **EP 1 525 875 A1**
(43) Date de publication de la demande: **27.04.2005**
(21) Numéro de dépôt: 04292274.0
(22) Date de dépôt: 22.09.2004
(51) Int. Cl.: A61K 7/00

(54) **Emulsion H/E cosmetique ou dermatologique de pH stable**

(30) Priorité: 23.10.2003 FR 0350724
(71) Demandeur: L'OREAL, 75008 Paris (FR)
(72) Inventeur: Senee, Jerome, 91510 Lardy (FR)
(74) Mandataire: Rasson, Catherine

(57) **Abrégé**

La présente invention se rapporte à une composition cosmétique et/ou dermatologique sous forme d'émulsion huile-dans-eau susceptible d'être obtenue par inversion de phase, dans laquelle la taille moyenne des globules d'huile va de 50 nm à 1000 nm, caractérisée par le fait qu'elle contient au moins un bromure d'alkyl-triméthylammonium.

L'incorporation d'un bromure d'alkyl-triméthylammonium et notamment du bromure de myristyl-triméthylammonium permet d'obtenir une émulsion qui, tout en ayant une bonne protection bactériologique, présente l'avantage d'avoir un pH qui reste stable au cours du temps, même à température au-dessus de la température ambiante.

Les compositions de l'invention peuvent être utilisées dans de nombreuses applications cosmétiques et dermatologiques. Elles peuvent être également utilisées pour imprégner un support et constituer une lingette pour la peau.

## Description

La présente invention se rapporte à une composition cosmétique et/ou dermatologique sous forme d'émulsion huile-dans-eau ultrafine susceptible d'être obtenue par inversion de phase, de pH stable grâce à l'incorporation d'un bromure d'alkyl-triméthylammonium. L'invention se rapporte aussi aux utilisations de la dite composition, notamment dans le domaine cosmétique, telle quelle ou sous forme d'article imprégné de ladite composition.

Il est courant d'utiliser dans le domaine cosmétique des émulsions, notamment des émulsions du type huile-dans-eau (phase huileuse dispersée dans une phase aqueuse constituant la phase continue) qui apportent lors de l'application sur la peau, un toucher doux et peu gras.

Les émulsions huile-dans-eau sont stabilisées généralement par des tensioactifs émulsionnants du type huile-dans-eau, qui, grâce à leur structure amphiphile, se placent à l'interface phase huileuse / phase aqueuse, et stabilisent ainsi les gouttelettes d'huile dispersées. Malgré la présence d'émulsionnants, les émulsions peuvent avoir tendance à se déphaser (séparation des phases aqueuse et huileuse avec relargage d'huile). L'augmentation de la teneur en émulsionnant permet le plus souvent d'améliorer la stabilité de l'émulsion. Toutefois, la présence d'émulsionnants à de fortes concentrations conduit à des problèmes d'inconfort de la composition obtenue lors de l'application, comme par exemple un toucher rêche ou un toucher collant ou poisseux, ainsi qu'à des problèmes d'innocuité vis à vis de la peau, des yeux et du cuir chevelu du fait d'une quantité importante d'émulsionnants.

Pour résoudre les problèmes de stabilité des émulsions H/E classiques, on a proposé de réaliser des émulsions H/E spécifiques dites "ultrafines", dans lesquelles la taille moyenne des globules constituant la phase huileuse est comprise dans des limites bien déterminées, à savoir entre 50 et 1000 nm, lesdites émulsions ultrafines de type H/E étant elles-mêmes obtenues généralement selon une technique d'émulsification par inversion de phase.

Cette technique est, dans son principe, bien connue de l'homme de l'art et est notamment décrite dans les articles "Phase Inversion Emusification", par Th. Fôrster et al, paru dans Cosmetics & Toiletries, vol. 106, Décembre 1991, pp 49-52 ; «Application of the phase-inversion-temperature method to the emulsification of cosmetics» par T. MITSUI et al, paru dans American Cosmetics and Perfumery, vol 87, Décembre 1972. Le principe de cette technique, tel que décrit actuellement, est le suivant : on prépare une émulsion E/H (introduction de la phase aqueuse dans la phase huileuse) à une température qui doit être supérieure à la température d'inversion de phase du système (en Anglais : Phase Inversion Température ou PIT), c'est à dire la température à laquelle l'équilibre entre les propriétés hydrophile et lipophile du ou des émulsionnants mis en ceuvre est atteint ; à température élevée, c'est-à-dire supérieure à la température d'inversion de phase (>PIT), l'émulsion est de type eau-dans-huile, et au cours de son refroidissement, cette émulsion s'inverse à la température d'inversion de phase, pour devenir une émulsion de type huile-dans-eau, et ceci en étant passée auparavant par un état de micro-émulsion.

Ces émulsions PIT sont le plus souvent très fluides. Elles présentent un aspect bleuté et peuvent être translucides. Toutefois, ces émulsions posent des problèmes de stabilité, et notamment de stabilité du pH qui a tendance à évoluer au cours du temps, en particulier quand la température dépasse la température ambiante (c'est-à-dire environ 25°C). En outre, leur protection bactériologique pose aussi un problème car elle est plus difficile que dans les émulsions classiques. Or, il est important d'avoir une composition dont le pH reste stable, et par ailleurs, de disposer d'une composition bien protégée sur le plan bactériologique.

Il subsiste donc le besoin d'émulsions H/E très fines, qui aient un pH stable dans le temps, et qui présentent une bonne protection bactériologique.

La demanderesse a découvert de manière surprenante que l'addition de bromures d'alkyl-triméthylammonium permettait d'obtenir une émulsion H/E ayant non seulement une bonne protection bactériologique, mais aussi un pH stable dans le temps.

Les émulsions H/E de l'invention présentent l'avantage d'être en outre très bien tolérées car les bromures d'alkyl-triméthylammonium sont des conservateurs et l'addition d'une petite quantité de ces conservateurs utilisés dans la composition selon l'invention suffit pour obtenir une bonne protection bactériologique. De plus, elles ont les avantages habituels des émulsions ultrafines, tels que la douceur, la fraîcheur et le confort à l'application.

La présente invention a donc pour objet une composition pour application topique sous forme d'émulsion huile-dans-eau comprenant une phase huileuse dispersée dans une phase aqueuse, dans laquelle la taille moyenne des globules d'huile va de 50 nm à 1000 nm (nanomètres), caractérisée par le fait qu'elle contient au moins un bromure d'alkyl-triméthylammonium. Cette composition peut constituer notamment une composition cosmétique ou dermatologique.

L'émulsion huile-dans-eau de l'invention est notamment susceptible d'être obtenue selon la technique d'émulsification par inversion de phase.

Du fait qu'elle est destinée à une application topique, la composition de l'invention contient un milieu physiologiquement acceptable. Par "milieu physiologiquement acceptable", on entend un milieu convenant à une application topique sur la peau ou les phanères, c'est-à-dire compatible avec la peau, les muqueuses, les lèvres, les cheveux et les ongles.

Les émulsions selon l'invention peuvent être plus ou moins fluides. Elles sont généralement fluides, et peuvent avoir alors une viscosité allant par exemple de 1 à 500 cPoises (1 à 500 mPa.s) et de préférence de 1 à 200 cPoises (1 à 200 mPa.s), la viscosité étant mesurée à température ambiante (25°C) avec un Rhéomat RM 180 (généralement au mobile 1 ou 2).

Comme indiqué ci-dessus, les bromures d'alkyl-triméthylammonium utilisés permettent d'obtenir des compositions dont le pH reste stable.

Aussi, la présente invention a encore pour objet l'utilisation d'au moins un bromure d'alkyl-triméthylammonium, pour stabiliser le pH d'une émulsion huile-dans-eau dans laquelle la taille moyenne des globules d'huile va de 50 nm à 1000 nm.

Selon une caractéristique essentielle des compositions conformes à la présente invention, la taille moyenne en nombre des particules liquides (ou globules) d'huile (ou phase huileuse) au sein de la phase aqueuse dispersante va de 50 nm à 1000 nm. De préférence, cette taille moyenne va de 70 nm à 350 nm et plus particulièrement de 70 à 300 nm.

En outre, les compositions selon l'invention sont caractérisées par une polydispersité très faible, c'est-à-dire que la taille des globules est très homogène. En règle générale, 90% des globules d'huile de l'émulsion huile-dans-eau ultrafine selon l'invention ont une taille moyenne en nombre allant de 70 à 350 nm. La différence de taille entre les plus grandes et les plus petites globules va généralement de 20 à 400 nm et préférentiellement de 30 à 200 nm, alors que dans les émulsions classiques (autres qu'émulsions PIT ou que micro-émulsions), la différence de taille entre les plus grandes et les plus petites globules est généralement supérieure à 1000 nm.

Comme bromures d'alkyl triméthylammonium, on peut par exemple citer le bromure de myristyl-triméthylammonium (nom CTFA : Myrtrimonium bromide), le bromure de dodécyl-triméthylammonium, le bromure d'hexadécyltriméthylammonium, et leurs mélanges tel que le mélange vendu sous la dénomination Cetrimide par la société FEF CHEMICALS. Il s'agit de préférence du bromure de myristyl-triméthylammonium.

La quantité de bromure(s) d'alkyl triméthylammonium dans la composition de l'invention doit être suffisante pour obtenir la stabilité souhaitée. En pratique, la quantité de bromure d'alkyl triméthylammonium peut aller par exemple de 0,05 à 3 % en poids de matière active, de préférence de 0,1 à 2 % en poids de matière active mieux de 0,15 à 1 % en poids de matière active par rapport au poids total de la composition.

La nature de la phase huileuse des émulsions selon l'invention n'est pas critique. Ainsi, la phase huileuse peut être constituée par tous les composés qui sont déjà connus de façon générale comme convenant pour la fabrication d'émulsions de type huile dans eau, notamment dans les domaines cosmétique ou dermatologique. En particulier, ces composés peuvent être choisis, seuls ou en mélanges, parmi les huiles d'origine végétale, animale ou minérale, les cires naturelles ou synthétiques, et les différents autres corps gras, ces huiles, notamment cosmétiques, et autres corps gras devant être physiologiquement acceptables.

La phase huileuse contient habituellement au moins une huile. Parmi les huiles pouvant rentrer dans la composition de la phase huileuse, on peut notamment citer :
- les huiles minérales telles que l'huile de paraffine et l'huile de vaseline ;
- les huiles hydrocarbonées d'origine animale telles que le perhydrosqualène ;
- les huiles hydrocarbonées d'origine végétale telles que l'huile d'amande douce, l'huile d'avocat, l'huile de ricin, l'huile de coriandre, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore, l'huile de seigle, les triglycérides des acides caprylique/caprique comme ceux vendus par la société Stearineries Dubois ou ceux vendus sous les dénominations Miglyol 810, 812 et 818 par la société Dynamit Nobel ;
- les huiles de synthèse, telles que l'huile de purcellin, les esters d'acide gras comme le myristate de butyle, le myristate d'isopropyle, le myristate de cétyle, le palmitate d'isopropyle, le palmitate d'ethyl-2 hexyle, l'adipate d'isopropyle, l'adipate d'éthylhéxyle, le stéarate de butyle, le stéarate d'héxadécyle, le stéarate d'isopropyle, le stéarate d'octyle, le stéarate d'isocétyle, l'oléate de décyle, le laurate d'héxyle, le dicaprylate de propylène glycol et les esters dérivés d'acide lanolique tels que le lanolate d'isopropyle, le lanolate d'isocétyle, le dioctylcyclohexane, les isoparaffines et les poly-α-oléfines.
- les éthers tels que l'éther dicaprylique (nom CTFA : Dicaprylyl ether) ; les benzoates d'alcools gras en C₁₂-C₁₅ (Finsolv TN de FINETEX) ;
- les hydrocarbures substantiellement linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam® ;
- les alcools gras comportant de 8 à 26 atomes de carbone, tels que les alcools laurique, cétylique, myristique, stéarylique, palmitique, oléique, linoléique, le 2-octyldodécanol, et leurs mélanges tels que l'alcool cétéarylique (mélange d'alcool cétylique et d'alcool stéarylique) ;
- les acétylglycérides ;
- les octanoates et décanoates d'alcools et de polyalcools tels que ceux de glycol et de glycérol ; les ricinoléates d'alcools et de polyalcools tels que ceux de cétyle ;
- les huiles fluorées et perfluorées ;
- la lanoline ; la lanoline hydrogénée ; la lanoline acétylée ;
- les huiles de silicone, volatiles ou non, comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, les polyméthyl-phénylsiloxanes.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

On peut utiliser une huile ou un mélange de plusieurs des huiles indiquées ci-dessus.

Selon un mode particulier de réalisation de l'invention, la composition comprend au moins une huile choisie parmi les huiles minérales, les esters d'acide gras, et leurs mélanges.

Selon un autre mode particulier de réalisation de l'invention, la composition comprend au moins un émollient choisi parmi les huiles indiquées ci-dessus, et plus particulièrement le palmitate d'éthyl-2 hexyle, l'éther dicaprylique, et leurs mélanges.

De manière classique, la phase aqueuse peut contenir, outre l'eau, un ou plusieurs solvants hydrosolubles choisis parmi les polyols (ou alcools polyhydriques), les alcool(s) inférieur(s) hydrosoluble(s) et leurs mélanges. On entend par "alcool inférieur", un alcool comportant de 1 à 8 atomes de carbone. Comme polyols, on peut citer par exemple la glycérine ; les glycols comme le propylène glycol, le butylène glycol ; le sorbitol ; les sucres tels que le glucose, le fructose, le maltose, le lactose, le sucrose ; et leurs mélanges. Comme alcools inférieurs, on peut citer par exemple l'éthanol, l'isopropanol, le butanol et leurs mélanges.

De façon préférentielle, les émulsions huile-dans-eau conformes à l'invention comportent :
(i) de 50 à 98 % en poids, plus préférentiellement de 70 à 96 % en poids de phase aqueuse, par rapport au poids total de la composition ;
(ii) de 2 à 50 % en poids, plus préférentiellement de 4 à 30 % en poids de phase huileuse, par rapport au poids total de la composition.

Les émulsions conformes à l'invention contiennent en outre généralement un ou plusieurs tensioactifs émulsionnants. Elles peuvent en outre contenir des co-émulsionnants spécifiques dont le rôle est, lors de la préparation de l'émulsion, de diminuer de manière substantielle la quantité d'agents tensioactifs émulsionnants, nécessaire à la réalisation de l'émulsion.

Les émulsionnants utilisables dans la présente invention sont préférentiellement choisis parmi les composés non-ioniques constitués d'une part d'un reste lipophile choisi par exemple parmi les fonctions alkyle ou acyle en C₆-C₃₀ et d'autre part d'un reste hydrophile choisi par exemple parmi les groupements polyols (glycérol, glycol, glucose) et les éthers de polyols. Leur balance HLB (« Hydrophilic Lipophilic Balance » ou balance hydrophile lipophile) peut aller par exemple de 9 à 18.

On peut citer en particulier comme émulsionnants non-ioniques préférentiellement utilisés dans la présente invention, les produits d'addition d'oxyde d'éthylène avec des alcools gras ayant 6 à 30 atomes de carbone ou avec des esters partiels de polyols ayant 3 à 16 atomes de carbone et d'acides gras ayant 14 à 22 atomes de carbone, et leurs mélanges.

Ainsi, comme produits d'addition d'oxyde d'éthylène avec des alcools gras, on peut utiliser un émulsionnant répondant à la formule (III) :

R-(O-CH₂-CH₂)ₙ-OH (III)

dans laquelle R représente un résidu hydrocarboné saturé ou insaturé, ramifié ou linéaire, ayant de 8 à 30 atomes de carbone et n représente un nombre allant de 8 à 50, préférentiellement de 8 à 30. On peut citer par exemple les produits d'addition d'oxyde d'éthylène avec l'alcool béhénylique, comme par exemple Beheneth-9 (9 moles d'oxyde d'éthylène) et Beheneth-10 (10 moles d'oxyde d'éthylène) ; ceux obtenus avec l'alcool cétylique ou avec l'alcool stéarylique ou avec leurs mélanges, notamment ceux comportant 12 à 20 moles d'oxyde d'éthylène, comme par exemple ceux obtenus à partir du mélange d'alcool cétylique et d'alcool stéarylique (alcool cétéarylique) comme Ceteareth-12, Ceteareth-13, Ceteareth-14, Ceteareth-15, Ceteareth-16, Ceteareth-17, Ceteareth-18, Ceteareth-20 ; ceux obtenus à partir de l'alcool cétylique comme Ceteth-14, Ceteth-15, Ceteth-16, Ceteth-20 ; ceux obtenus à partir de l'alcool stéarylique comme Steareth-13, Steareth-14, Steareth-15, Steareth-16, Steareth-20, et les mélanges de ces différents émulsionnants.

Les produits d'addition d'oxyde d'éthylène avec des esters partiels de polyols et d'acides gras peuvent être obtenus par éthoxylation d'esters partiels d'acides gras du glycérol ou de mono ou de di-esters d'acides gras du sorbitol. On peut utiliser par exemple les produits d'addition de 4 à 20 moles d'oxyde d'éthylène sur un ou plusieurs esters partiels de glycérol. Par esters partiels de glycérol, on entend par exemple les mélanges de mono, di- et tri-glycérides d'acides gras en C₁₀-C₂₀ obtenus par estérification d'une mole de glycérol par 1 ou 2 moles d'un acide gras en C₁₀-C₂₀.

En outre, les émulsions selon l'invention peuvent comprendre en plus un ou plusieurs co-émulsionnants. Ce ou ces co-émulsionnants peuvent être choisis par exemple parmi les alcools gras en C₁₆-C₂₂ ou les esters de polyols en C₃-C₆ avec des acides gras en C₁₄-C₂₂, et leurs mélanges. Comme co-émulsionnant, on peut citer par exemple l'alcool cétylique, l'alcool stéarylique, l'alcool cétéarylique (mélange d'alcool cétylique et d'alcool stéarylique), le stéarate de glycéryle, et leurs mélanges.

On peut utiliser par exemple comme mélange contenant émulsionnant et co-émulsionnant le mélange commercialisé sous la dénomination Emulgade SEV par la société Cognis contenant du Ceteareth-20, du Ceteareth-12, de l'huile de palme hydrogénée et de l'alcool cétéarylique.

La quantité d'émulsionnants et de co-émulsionnants dans la composition de l'invention va généralement de 0,05 à 30 % en poids, de préférence de 0,5 à 20 % en poids et mieux de 1 à 10 % en poids par rapport au poids total de la composition.

La composition selon l'invention peut aussi contenir tout adjuvant ou additif habituellement utilisé dans les domaines considérés et notamment dans les domaines cosmétique ou dermatologique.

Parmi les adjuvants classiques susceptibles d'être contenus dans la phase aqueuse et/ou dans la phase huileuse des émulsions conformes à l'invention (selon le caractère hydrosoluble ou liposoluble de ces adjuvants), on peut citer notamment les gélifiants et les épaississants ioniques ou non ioniques ; les actifs cosmétiques ou dermatologiques, hydrophiles ou lipophiles ; les parfums ; les conservateurs ; les séquestrants (EDTA) ; les pigments ; les nacres ; les charges minérales ou organiques telles que le talc, le kaolin, les poudres de silice ou de polyéthylène ; les colorants solubles ; les filtres solaires ; des agents alcanisants ou acidifiants ; les composés améliorant la douceur et le glissant de la composition à l'application, tels que les cétyldiméthicones, et leurs mélanges. Les quantités de ces différents adjuvants sont celles classiquement utilisées dans le domaine considéré, et par exemple de 0,01 à 20 % du poids total de la composition.

Le choix des actifs cosmétiques ou dermatologiques pouvant être utilisés dans la composition de l'invention dépend de la finalité de cette composition. Comme actifs utilisables dans l'invention, on peut citer par exemple les antibactériens comme l'octopirox, le triclosan et le triclocarban ; les agents kératolytiques et anti-vieillissement comme les hydroxyacides (α-hydroxyacides et β-hydroxyacides) tels que l'acide lactique, l'acide glycolique, l'acide citrique, l'acide salicylique et leurs dérivés ; les huiles essentielles ; les vitamines et en particulier le rétinol (vitamine A), l'acide ascorbique (vitamine C), le tocophérol (vitamine E), la niacinamide (vitamine PP ou B3), le panthénol (vitamine B5) et leurs dérivés (esters par exemple) ; les co-enzymes et en particulier le co-enzyme Q10 ou ubiquinone ; les enzymes telles que par exemple les lipases, les protéases, les phospholipases, les cellulases, les peroxydases notamment les lactoperoxydases, les catalases, les superoxyde dismutases et les extraits végétaux contenant les enzymes précitées ; les levures telles que les *Saccharomyces Cerevisiae ;* les stéroïdes ; les anti-oxydants et anti-radicaux libres ; les hydratants tels que les polyols (glycérine, sorbitol, sucres), les hydrolysats de protéine, l'urée et les mélanges en contenant ; les amincissants comme la caféine ; les agents anti-élastase et anticollagénase ; les agents de traitement des peaux grasses ; et leurs mélanges.

Comme dérivés d'acide salicylique, on peut citer notamment les acides n-octanoyl-5-salicylique (nom CTFA : Capryloyl Salicylic Acid), n-décanoyl-5-salicylique, n-dodécanoyl-5-salicylique, n-octyl-5-salicylique, n-heptyloxy-5-salicylique, 5-tert-octylsalicylique, 5-butoxy-salicylique, 5-éthoxy-salicylique, 5-méthoxysalicylique, 5-propoxysalicylique, 5-méthylsalicylique, 5-éthylsalicylique, 5-propylsalicylique, et leurs mélanges. Ces acides peuvent être éventuellement salifiés par une base.

Comme exemples de stéroïdes, on peut citer par exemple la déhydroépiandrostérone (ou DHEA), ainsi que (1) ses précurseurs et dérivés biologiques, en particulier les sels et esters de DHEA, tels que le sulfate et le salicylate de DHEA, la 7-hydroxy DHEA, la 7-céto DHEA, les esters de 7-hydroxy et 7-céto DHEA, notamment la 3-beta-acétoxy-7-oxo DHEA, et (2) ses précurseurs et dérivés chimiques, en particulier les sapogénines telles que la diosgénine ou l'hécogénine, et/ou leurs dérivés tels que l'acétate d'hécogénine, et/ou les extraits naturels en contenant et notamment les extraits de Dioscorées, tels que l'igname sauvage (Wild Yam).

Les filtres solaires (ou filtres U.V.) éventuellement présents dans la composition peuvent être choisis parmi les filtres organiques, les filtres physiques et leurs mélanges.

Comme filtres solaires chimiques utilisables dans la composition de l'invention, la composition de l'invention peut comprendre tous les filtres UVA et UVB utilisables dans le domaine cosmétique.

Comme filtres UVB, on peut citer par exemple :
(1) les dérivés de l'acide salicylique, en particulier le salicylate d'homomenthyle et le salicylate d'octyle ;
(2) les dérivés de l'acide cinnamique, en particulier le p-méthoxycinnamate de 2-éthylhexyle, commercialisé par la société Givaudan sous la dénomination Parsol MCX ;
(3) les dérivés de β,β'-diphénylacrylate liquides, en particulier l'α-cyano-β,β' diphénylacrylate de 2-éthylhexyle ou octocrylène, commercialisé par la société BASF sous la dénomination UVINUL N539 ;
(4) les dérivés de l'acide p-aminobenzoïque ;
(5) le 4-méthyl benzylidène camphre commercialisé par la société Merck sous la dénomination EUSOLEX 6300 ;
(6) l'acide 2-phénylbenzimidazole 5-sulfonique commercialisé sous la dénomination EUSOLEX 232 par la société Merck ;
(7) les dérivés de 1,3,5-triazine, en particulier :
   - la 2,4,6-tris[p-(2'-éthylhexyl-1'-oxycarbonyl)anilino]-1,3,5-triazine commercialisé par la société BASF sous la dénomination UVINUL T150, et
   - le dioctyl butamido triazone commercialisé par la société Sigma 3V sous la dénomination UVASORB HEB ;
(8) les mélanges de ces filtres.

Comme filtres UVA, on peut citer par exemple :
(1) les dérivés de dibenzoylméthane, en particulier le 4-(ter.-butyl) 4'-méthoxy dibenzoylméthane commercialisé par la société Givaudan sous la dénomination PARSOL 1789 ;
(2) l'acide benzène 1,4 [di(3-méthylidènecampho-10-sulfonique)] éventuellement sous forme partiellement ou totalement neutralisée, commercialisé sous la dénomination MEXORYL SX par la société Chimex.
(3) les dérivés de benzophénone, par exemple :
   - la 2,4-dihydroxybenzophénone (benzophénone-1) ;
   - la 2,2',4,4'-tétra-hydroxybenzophénone (benzophénone-2) ;
   - la 2-hydroxy-4-méthoxy-benzophénone (benzophénone-3), commercialisé sous la dénomination UVINUL M40 par la société BASF ;
   - l'acide 2-hydroxy-4-méthoxy-benzophénone-5-sulfonique (benzophénone-4) ainsi que sa forme sulfonate (benzophénone-5), commercialisé par la société BASF sous la dénomination UVINUL MS40 ;
   - la 2,2'-dihydroxy-4,4'-diméthoxy-benzophénone (benzophénone-6) ;
   - la 5-chloro-2-hydroxybenzophénone (benzophénone-7) ;
   - la 2,2'-dihydroxy-4-méthoxy-benzophénone (benzophénone-8) ;
   - le sel disodique du diacide 2,2'-dihydroxy-4,4'-diméthoxy-benzophénone-5,5'-disulfonique (benzophénone-9) ;
   - la 2-hydroxy-4-méthoxy-4'-méthyl-benzophénone (benzophénone-10) ;
   - la benzophénone-11 ;
   - la 2-hydroxy-4-(octyloxy)benzophénone (benzophénone-12).
(4) les dérivés silanes ou les polyorganosiloxanes à groupement benzophénone ;
(5) les anthranilates, en particulier l'anthranilate de menthyle commercialisé par la société Haarman & Reiner sous la dénomination NEO HELIOPAN MA ;
(6) les composés comportant par molécule au moins deux groupes benzazolyle ou au moins un groupe benzodiazolyle, en particulier l'acide 1,4-bis-benzimidazolyl-phenylène-3,3',5,5'-tétrasulfonique ainsi que ses sels commercialisés par la société Haarman & Reimer ;
(7) les dérivés siliciés de benzimidazolyl-benzazoles N-substitués ou de benzofuranyl-benzazoles, et en particulier :
   - le 2-[1-[3-[1,3,3,3-tétraméthy)-1-[(triméthylsilyl)oxy]disiloxanyl]propyl]-1H-benzimidazol-2-yl]-benzoxazole ;
   - le 2-[1-[3-[1,3,3,3-tétraméthyl-1-[(triméthylsilyl)oxy]disiloxanyl]propyl]-1H-benzimidazol-2-yl]-benzothiazole ;
   - le 2-[1-(3-trimethylsilanyl-propyl)-1H-benzimidazol-2-yl]-benzoxazole ;
   - le 6-méthoxy-1,1'-bis-(3-triméthylsilanyl-propyl)-1H,1'H-[2,2']bibenzimidazolylbenzoxazole ;
   - le 2-[1-(3-trimethylsilanyl-propyl)-1H-benzimidazol-2-yl]-benzothiazole ; qui sont décrits dans la demande de brevet EP-A-1 028 120 ;
(8) les dérivés de triazine, et en particulier la 2,4-bis [4-(2-éthyl-hexyloxy)-2-hydroxy]-phényl]-6-(4-méthoxy-phényl)-1,3,5-triazine commercialisé par la société Ciba Geigy sous la dénomination TINOSORB S, et le 2,2'-méthylènebis-[6-(2H benzotriazol-2-yl)4-(1,1,3,3-tétraméthylbutyl)phénol] commercialisé par la société Ciba Geigy sous la dénomination TINOSORB M ;
(9) leurs mélanges.

On peut aussi utiliser un mélange de plusieurs de ces filtres et un mélange de filtres UVB et de filtres UVA et aussi des mélanges avec des filtres physiques.

Comme filtres physiques, on peut citer les oxydes de titane (dioxyde de titane amorphe ou cristallisé sous forme rutile et/ou anatase), de zinc, de fer, de zirconium, de cérium ou leurs mélanges. Ces oxydes métalliques peuvent être sous forme de particules ayant une taille micrométrique ou nanométrique (nano-pigments). Sous forme de nano-pigments, les tailles moyennes des particules vont par exemple de 5 à 100 nm. On utilise de préférence dans la composition de l'invention des nano-pigments.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas ou substantiellement pas, altérées par l'addition envisagée.

Les émulsions selon l'invention sont obtenues généralement par un procédé d'inversion de phase. Ce procédé de préparation est caractérisé en ce que :
1) on mélange :
   (A) la phase huileuse, cette phase huileuse comportant au moins une huile et éventuellement d'autres huiles et corps gras et les adjuvants liposolubles stables à la température d'inversion de phase,
   (B) au moins un émulsionnant et éventuellement un co-émulsionnant,
   (D) la phase aqueuse, cette phase aqueuse comportant de l'eau et éventuellement les solvants hydrosolubles et les adjuvants hydrosolubles stables à la température d'inversion de phase,
   afin d'obtenir une émulsion classique,
(2) on chauffe cette émulsion à une température située à l'intérieur ou au-dessus du domaine d'inversion de phase, ou bien on prépare l'émulsion directement à une telle température, puis
(3) on refroidit l'émulsion et on ajoute le bromure d'alkyl triméthylammonium et éventuellement les adjuvants ou actifs instables à la température d'inversion de phase, tels que par exemple les parfums.

On peut aussi utiliser d'autres modes opératoires, et par exemple n'ajouter qu'une partie de la phase aqueuse lors de la préparation de l'émulsion (par exemple 10 à 30 % de la phase aqueuse) et diluer ensuite l'émulsion avec le reste de phase aqueuse contenant le ou les bromures d'alkyl triméthylammonium après le refroidissement.

Le domaine de température d'inversion de phase peut être observé visuellement en observant les changements de transparence et d'opacité de l'émulsion, l'émulsion étant opaque quand elle est au départ sous forme d'émulsion E/H, puis transparente quand elle devient une microémulsion, de nouveau opaque quand elle est sous forme d'émulsion H/E, puis transparente de nouveau quand elle est refroidie en dessous de la température d'inversion de phase. Ce domaine de température peut aussi être établi, pour une composition donnée, en mesurant la conductibilité d'un échantillon de la composition que l'on chauffe. Lorsque l'on atteint le domaine d'inversion de phase, la conductibilité de l'émulsion augmente de façon très rapide : dans le domaine d'inversion de phase, on peut observer une augmentation de la conductibilité d'environ 50 micro- siemens par centimètre sur un intervalle de température de 5 à 15°C, tandis qu'elle ne sera que d'environ 5 micro-siemens par centimètre sur un intervalle de température équivalent en dehors du domaine d'inversion de phase.

Les compositions selon l'invention peuvent se présenter en particulier sous forme de lotion ou de lait plus ou moins fluide. Elles peuvent être utilisées telles quelles ou en aérosol ou sous forme de spray.

Les compositions de l'invention peuvent constituer notamment des produits de soin, de protection et/ou d'hygiène pour la peau du visage et/ou du corps, le cuir chevelu ou des muqueuses, tels que des sprays de protection, de traitement ou de soin pour le visage, pour les mains ou pour le corps, des laits ou lotions corporels de protection ou de soin de la peau, du cuir chevelu ou des muqueuses ou pour le démaquillage et/ou le nettoyage de la peau et/ou des lèvres. Elles peuvent être utilisées par exemple pour l'hydratation et/ou le raffermissement de la peau et/ou pour nourrir la peau.

Les compositions selon l'invention peuvent constituer aussi des produits solaires pour protéger la peau du corps et/ou du visage des effets néfastes du soleil ou des U.V., et également des produits pour le maquillage des matières kératiniques et notamment de la peau ou des lèvres.

Ainsi, la présente invention a pour objet l'utilisation cosmétique de la composition telle que définie ci-dessus, pour le traitement, la protection, le soin, le démaquillage et/ou le nettoyage de la peau et/ou des lèvres, et/ou pour le maquillage de la peau et/ou des lèvres.

La présente invention a encore pour objet un procédé de traitement cosmétique de la peau et/ou des lèvres, caractérisé par le fait que l'on applique sur la peau, et/ou les lèvres, une composition telle que définie ci-dessus. Le type de traitement peut varier notamment en fonction du ou des actifs présents dans la composition.

Selon un autre mode de réalisation de l'invention, les compositions selon l'invention peuvent être utilisées pour imprégner un substrat insoluble dans l'eau afin de constituer un article à appliquer sur la peau, tel qu'une lingette. Le substrat insoluble dans l'eau peut être choisi dans le groupe comprenant des matériaux tissés, des matériaux non-tissés, des mousses, des éponges, des ouates, en feuilles, boules ou films. Il peut s'agir notamment d'un substrat non tissé à base de fibres d'origine naturelle (lin, laine, coton, soie) ou d'origine synthétique (dérivés de cellulose, viscose, dérivés polyvinyliques, polyesters comme téréphtalate de polyéthylène, polyoléfines comme polyéthylène ou polypropylène, polyamides comme le Nylon, dérivés acryliques). Les non tissés sont décrits de façon générale dans RIEDEL « Nonwoven Bonding Methods & Materials », Nonwoven World (1987). Ces substrats sont obtenus selon les procédés usuels de la technique de préparation des non-tissés. Selon un mode particulier de réalisation de l'invention, le substrat insoluble peut contenir un au moins des composés et notamment des conservateurs de l'invention, fixé au support par les moyens connus de greffage des agents biocides sur les fibres.

Ce substrat peut comporter une ou plusieurs couches ayant des propriétés identiques ou différentes et avoir des propriétés d'élasticité, de douceur et autres appropriées à l'usage recherché. Les substrats peuvent comporter par exemple deux parties ayant des propriétés d'élasticité différentes comme décrit dans le document WO-A-99/13861 ou comporter une seule couche avec des densités différentes comme décrit dans le document WO-A-99/25318 ou comporter deux couches de textures différentes comme décrit dans le document WO-A-98/18441.

Le substrat peut avoir toute taille et toute forme appropriées au but recherché. Il a généralement une surface comprise entre 0,005 m et 0,1 m², de préférence entre 0,01 m² et 0,05 m². Il se présente de préférence sous la forme de lingettes rectangulaires ou de compresses rondes.

L'article final comportant le substrat et la composition d'imprégnation est généralement à l'état humide, avec un taux d'imprégnation de la composition allant par exemple de 200 à 1000 %, et de préférence de 250 à 350 % en poids de composition par rapport au poids de substrat. Les techniques d'imprégnation des substrats par des compositions sont bien connues dans ce domaine et sont toutes applicables à la présente invention. En général, la composition d'imprégnation est ajoutée au substrat par une ou plusieurs techniques comprenant l'immersion, l'enduction, la vaporisation, etc.

Il est aussi possible de constituer un article (ou lingette) présenté à l'état sec soit en éliminant l'eau de la composition après son imprégnation sur le substrat, soit en imprégnant le substrat avec une composition sous forme sèche à l'état de poudre, granule ou film, par tous les moyens de réalisation connus comme le soudage et le collage de multicouches par voies thermiques ou ultrasons. Dans ce dernier mode de réalisation, la composition est séchée par tout moyen connu : atomisation, lyophilisation ou autre procédé analogue.

On peut ainsi obtenir selon l'utilisation envisagée, des lingettes humides ou des lingettes sèches. Les lingettes humides peuvent être utilisées telles quelles alors que les lingettes sèches sont mouillées avant utilisation.

L'invention a donc aussi pour objet un article comportant (A) un substrat insoluble dans l'eau, (B) une composition telle que définie ci-dessus, ajoutée ou imprégnée sur le substrat, ainsi que les utilisations dudit article dans le domaine cosmétique ou dermatologique, en particulier pour le soin (nourrir, raffermir hydrater), le nettoyage et/ou le démaquillage de la peau.

Cet article peut constituer notamment des lingettes de soin et/ou de traitement de la peau, et/ou des lingettes de nettoyage et/ou de démaquillage de la peau et/ou des yeux.

L'invention a aussi pour objet l'utilisation cosmétique d'un article tel que défini ci-dessus, pour le soin, le nettoyage et/ou le démaquillage de la peau et:ou des yeux.

Les exemples indiqués ci-dessous permettront de mieux comprendre l'invention sans toutefois présenter un caractère limitatif. Les quantités indiquées sont en % en poids sauf mention contraire. Les noms sont en noms chimiques et en noms CTFA selon les composés.

### Exemple 1 selon l'invention : lait fluide

| *Phase A* | |
|---|---|
| Huile minérale | 5 % |
| Ether di-caprylique (Cetiol OE de la société Cognis) | 3 % |
| Mélange de Ceteareth-20, Ceteareth-12, huile de palme (Emulgade SEV de la société Cognis) | 3 % |
| Ceteareth-20 (Emulgin B2 de la société Cognis) | 1 % |
| Palmitate d'éthyl-2 hexyle | 3 % |
| Cetyldimethicone (Abil Wax 9801 de la société Goldschmidt) | 0,1 % |
| | |

| *Phase B* | |
|---|---|
| Glycérine | 5 % |
| Eau distillée | 15 % |
| | |

| *Phase C* | |
|---|---|
| Huile de palme | 0,2 % |
| Parfum | 0,4 % |
| | |

| *Phase D* | |
|---|---|
| Bromure de myristyl triméthylammonium | 0,2 % |
| Eau distillée | qsp 100 % |

Mode opératoire : On chauffe séparément à 70 °C les phases A et B sous agitation, et on les homogénéise. Sous agitation lente, on verse la phase B sur la phase A et on chauffe le mélange jusqu'à la température d'inversion de phase (PIT) qui est à une température d'environ 60 à 80°C. L'émulsion eau-dans-huile obtenue devient quasi-transparente lors du chauffage. On arrête le chauffage quand l'émulsion est devenue blanche et on verse la phase C dans l'émulsion en gardant une agitation lente, puis, dès que l'émulsion devient transparente lors du refroidissement, on dilue en ajoutant la phase D.

On obtient une émulsion huile-dans-eau fluide (viscosité environ 1 cPoise), légèrement bleutée. Après stockage à différentes températures, le pH est resté stable comme le montre le tableau ci-dessous :

| Stockage pendant deux mois à | Variation du pH |
|---|---|
| Température ambiante (environ 25°C) | + 0,01 |
| 37°C | + 0,07 |
| 45°C | + 0,25 |

En outre, la protection microbienne est bonne.

Cette émulsion peut être utilisée telle quelle ou en spray ou imprégnée sur un support non tissé pour constituer une lingette à appliquer sur le visage ou le corps, et elle peut être utilisée en vue par exemple de nourrir, hydrater et raffermir la peau.

### Exemple 2 comparatif :

| *Phase A* | |
|---|---|
| Huile minérale | 5 % |
| Ether di-caprylique (Cetiol OE de la société Cognis) | 3 % |
| Mélange de Ceteareth-20, Ceteareth-12, huile de palme hydrogénée et alcool cétéarylique (Emulgade SEV de la société Cognis) | 3 % |
| Ceteareth-20 (Emulgin B2 de la société Cognis) | 1 % |
| Palmitate d'éthyl-2 hexyle | 3 % |
| Cetyldimethicone (Abil Wax 9801 de la société Goldschmidt) | 0,1 % |
| | |

| *Phase B* | |
|---|---|
| Glycérine | 5 % |
| Eau distillée | 15 % |
| Methylparaben | 0,2 % |
| | |

| *Phase C* | |
|---|---|
| Huile de palme | 0,2 % |
| Parfum | 0,4 % |
| | |

| *Phase D* | |
|---|---|
| Imidazolidinyl urée (Germall 115) | 0,3 % |
| Eau distillée | qsp 100 % |

Le mode opératoire est identique à celui de l'exemple 1 selon l'invention.

Dans cet exemple, on a remplacé le bromure de myristyl triméthylammonium de l'exemple 1 par un autre conservateur (Germall 115).

Il a été constaté que le pH évoluait au cours du temps comme le montre le tableau ci-dessous :

| Stockage pendant deux mois à | Variation du pH |
|---|---|
| Température ambiante (environ 25°C) | - 0,57 |
| 37°C | - 1,11 |
| 45°C | - 1,28 |

En outre, la protection microbienne se révèle insuffisante.

### Exemple 3 comparatif :

| *Phase A* | |
|---|---|
| Huile minérale | 5 % |
| Ether di-caprylique (Cetiol OE de la société Cognis) | 3 % |
| Mélange de Ceteareth-20, Ceteareth-12, huile de palme hydrogénée et alcool cétéarylique (Emulgade SEV de la société Cognis) | 3 % |
| Ceteareth-20 (Emulgin B2 de la société Cognis) | 1 % |
| Palmitate d'éthyl-2 hexyle | 3 % |
| Cetyldimethicone (Abil Wax 9801 de la société Goldschmidt) | 0,1 % |
| | |

| *Phase B* | |
|---|---|
| Glycérine | 5 % |
| Eau distillée | 15 % |
| Methylparaben | 0,2 % |
| | |

| *Phase C* | |
|---|---|
| Huile de palme | 0,2 % |
| Parfum | 0,4 % |
| | |

| *Phase D* | |
|---|---|
| Chlorphenesine | 0,25 % |
| Eau distillée | qsp 100 % |

Le mode opératoire est identique à celui de l'exemple 1 selon l'invention.

Dans cet exemple, on a remplacé le bromure de myristyl triméthylammonium de l'exemple 1 par un autre conservateur (chlorphenesine).

Il a été constaté que le pH évoluait au cours du temps comme le montre le tableau ci-dessous :

| Stockage pendant deux mois à | Variation du pH |
|---|---|
| Température ambiante (environ 25°C) | - 0,24 |
| 37°C | - 0,22 |
| 45°C | - 0,65 |

En outre, la protection microbienne est insuffisante.

### Exemple 4 selon l'invention : lait fluide

| *Phase A* | |
|---|---|
| Cyclomethicone | 4,5 % |
| Ether di-caprylique (Cetiol OE de la société Cognis) | 3 % |
| Mélange de Ceteareth-20, Ceteareth-12, huile de palme hydrogénée et alcool cétéarylique (Emulgade SEV de la société Cognis) | 3% |
| Ceteareth-15 (Mergital CS15 de la société Cognis) | 1,5 % |
| Stéarate de glycéryle | 0,9 % |
| Parfum | 0,7 % |
| | |

| *Phase B* | |
|---|---|
| Glycérine | 5 % |
| Eau distillée | 15 % |
| Methyl paraben | 0,2 % |
| | |

| *Phase C* | |
|---|---|
| Alcool éthylique | 3 % |
| | |

| *Phase D* | |
|---|---|
| Bromure de myristyl triméthylammonium | 0,2 % |
| Eau distillée | qsp 100 % |

Le mode opératoire est le même que pour l'exemple 1.

On obtient une émulsion huile-dans-eau fluide (viscosité environ 1 cPoise, légèrement bleutée. Après stockage à différentes températures, le pH est resté stable comme le montre le tableau ci-dessous :

| Stockage pendant deux mois à | Variation du pH |
|---|---|
| Température ambiante (environ 25°C) | + 0,05 |
| 37°C | + 0,06 |
| 45°C | + 0,04 |

En outre, la protection microbienne est bonne.

Cette émulsion peut être utilisée telle quelle ou en spray ou imprégnée sur un support non tissé pour constituer une lingette à appliquer sur le visage ou le corps, et elle peut être utilisée en vue par exemple de nourrir, hydrater et raffermir la peau.

## Revendications

1. Composition pour application topique sous forme d'émulsion huile-dans-eau comprenant une phase huileuse dispersée dans une phase aqueuse, dans laquelle la taille moyenne des globules d'huile va de 50 nm à 1000 nm, **caractérisée par le fait qu'**elle contient au moins un bromure d'alkyltriméthylammonium.

2. Composition selon la revendication 1, **caractérisée par le fait qu'**elle est susceptible d'être obtenue selon la technique d'émulsification par inversion de phase.

3. Composition selon la revendication 1 ou 2, **caractérisée par le fait que** le bromure d'alkyl-triméthylammonium est choisi parmi le bromure de dodécyl-triméthylammonium, le bromure de myristyl-triméthylammonium, le bromure d'hexadécyl-triméthylammonium, et leurs mélanges.

4. Composition selon l'une quelconque des revendications précédentes, **caractérisée par le fait que** la quantité de bromure d'alkyl-triméthylammonium va de 0,05 à 3 % en poids et de préférence de 0,1 à 2 % en poids de matière active par rapport au poids total de la composition.

5. Composition selon l'une quelconque des revendications précédente, **caractérisée par le fait que** la phase huileuse contient au moins une huile choisie parmi les huiles minérales, les esters d'acides gras et leurs mélanges.

6. Composition selon l'une quelconque des revendications précédentes, **caractérisée par le fait que** la phase aqueuse représente de 50 à 98 % en poids et de préférence de 70 à 96 % en poids, par rapport au poids total de la composition.

7. Composition selon l'une quelconque des revendications précédentes, **caractérisée par le fait qu'**elle contient au moins un émulsionnant ayant un HLB de 9 à 18, choisi parmi les composés non-ioniques constitués d'une part d'un reste lipophile choisi parmi les fonctions alkyle ou acyle en C₆-C₃₀ et d'autre part d'un reste hydrophile choisi parmi les groupements polyols et les éthers de polyols.

8. Composition selon la revendication précédente, **caractérisée par le fait que** l'émulsionnant est choisi dans le groupe constitué par :
- les composés répondant à la formule (III) :
R-(O-CH₂-CH₂)ₙ-OH (III)
dans laquelle R représente un résidu hydrocarboné saturé ou insaturé, ramifié ou linéaire, ayant de 8 à 30 atomes de carbone et n représente un nombre allant de 8 à 50, préférentiellement de 8 à 30 ;
- les produits d'addition de 4 à 20 moles d'oxyde d'éthylène sur un ou plusieurs esters partiels de glycérol ;
- et leurs mélanges.

9. Composition selon la revendication précédente, **caractérisée par le fait que** l'émulsionnant est choisi parmi les produits d'addition d'oxyde d'éthylène avec l'alcool béhénylique, l'alcool cétylique, l'alcool stéarylique, et leurs mélanges.

10. Composition selon l'une quelconque des revendications précédentes, **caractérisée en ce qu'**elle contient en outre un co-émulsionnant choisi parmi les alcools gras en C₁₆-C₂₂ ou les esters partiels de polyols en C₃-C₆ avec des acides gras en C₁₄-C₂₂, et leurs mélanges.

11. Composition selon l'une quelconque des revendications 7 à 10, **caractérisée par le fait que** la quantité d'émulsionnants et de co-émulsionnants va généralement de 0,05 à 30 % en poids, de préférence de 0,5 à 20 % en poids par rapport au poids total de la composition.

12. Composition selon l'une quelconque des revendications précédentes, **caractérisée par le fait qu'**elle constitue une composition cosmétique ou dermatologique.

13. Composition selon l'une quelconque des revendications précédentes, **caractérisée par le fait qu'**elle constitue un produit de soin, de protection et/ou d'hygiène pour la peau du visage et/ou du corps, le cuir chevelu, les muqueuses.

14. Utilisation cosmétique d'une composition cosmétique selon l'une quelconque des revendications 1 à 11, pour le traitement, la protection, le soin, le démaquillage et/ou le nettoyage de la peau et/ou des lèvres, et/ou pour le maquillage de la peau et/ou des lèvres et/ou comme produit solaire.

15. Procédé de traitement cosmétique de la peau et/ou des lèvres, **caractérisé par le fait que** l'on applique sur la peau et/ou les lèvres, une composition cosmétique selon l'une quelconque des revendications 1 à 11.

16. Utilisation de la composition selon l'une quelconque des revendications 1 à 11 pour imprégner un substrat insoluble dans l'eau.

17. Article comportant (A) un substrat insoluble dans l'eau, et (B) une composition selon l'une quelconque des revendications 1 à 11, ajoutée ou imprégnée sur le substrat.

18. Article selon la revendication précédente, **caractérisé en ce que** le substrat est choisi dans le groupe comprenant les matériaux tissés, les matériaux non-tissés, les mousses, les éponges, les ouates.

19. Article selon la revendication 17 ou 18, **caractérisé en ce qu'**il constitue une lingette de soin et/ou de traitement de la peau ou une lingette de nettoyage et/ou de démaquillage de la peau et/ou des yeux.

20. Utilisation cosmétique de l'article selon la revendication 18 ou 19, pour le soin, le nettoyage et/ou le démaquillage de la peau et/ou des yeux.

21. Utilisation d'au moins un bromure d'alkyl-triméthylammonium, pour stabiliser le pH d'une émulsion huile-dans-eau dans laquelle la taille moyenne des globules d'huile va de 50 nm à 1000 nm.
